# EUROPEAN PATENT SPECIFICATION

(11) **EP 2 953 476 B1**
(45) Date of publication and mention of the grant of the patent: **01.01.2020**
(21) Application number: 14702635.5
(22) Date of filing: 05.02.2014
(51) Int. Cl.: A23C 9/123, C12N 1/20, C12R 1/225, A23C 9/152, A61K 35/747

(54) **USE OF SKIM MILK AS A CARRIER FOR A COMPOSITION COMPRISING LACTOBACILLUS**
VERWENDUNG VON MAGERMILCH ALS TRÄGER FÜR EINE ZUSAMMENSETZUNG MIT LACTOBACILLUS
UTILISATION DE LAIT ÉCRÉMÉ COMME SUPPORT DANS UNE COMPOSITION COMPORTANT DU LACTOBACILLUS

(30) Priority: 07.02.2013 EP 13154353
(43) Date of publication of application: 16.12.2015
(73) Proprietor: Novozymes A/S, 2880 Bagsværd (DK)
(72) Inventor: ROBINS, Karen Tracey, Sylvania New South Wales 2224 (AU); MAZOTTI, Judith, CH-3935 Bürchen (CH); GASSER, Kurt, CH-3904 Naters (CH)
(74) Representative: NZ EPO Representatives
(86) International application number: PCT/EP2014/052252
(87) International publication number: WO 2014/122175

(56) References cited:
- WO-A1-2007/073709
- WO-A1-2012/168468

## Description

The invention relates to a food composition comprising Lactobacillus, which is capable of aggregating Helicobacter pylori under physiological conditions, and a carrier, wherein the carrier comprises skim milk. The invention also relates to methods for producing such food compositions, uses thereof and uses of carriers.

### Background of the invention

Helicobacter pylori (H. pylori) is a bacterium of helical shape, which colonizes the stomach. In order to colonize the stomach, the bacteria enter the mucus, which covers the stomach's epithelial cell layer. H. pylori are also found on the inner surface of the stomach epithelial cells and occasionally inside epithelial cells. The cells are attached to the stomach wall by specific molecules, such as adhesines. In contrast, H. pylori has a limited stability in the acidic lumen and is secreted easily therefrom. In most individuals, an infection with H. pylori is harmless. However, acute infections can develop which are associated with various diseases, such as gastritis or ulcers. Contact of the epithelial layer with the bacteria is considered to be a requirement of the development of such infectious diseases.

It is known in the art that Lactobacillus has advantageous effects against H. pylori. Lactobacillus is a genus of specific bacteria, which are capable of converting lactose and other sugars into lactic acid.

US 5,716,615 discloses pharmaceutical compositions comprising Lactobacillus and other active ingredients. The composition is useful for treating disorders in the gastrointestinal tract. US 2005/0186190 A1 discloses a dietetic or pharmaceutical composition which comprises sphingomyelinase or Lactobacillus, which comprises sphingomyelinase. The composition is useful for the treatment of infections by H. pylori. WO2004/087891 A1 discloses specific strains of Lactobacillus, which are useful for preparing pharmaceutical or dietetic compositions for the treatment of infections of the gastrointestinal tract caused by H. pylori.

WO2005/060937 A1 discloses tablet-like formulations comprising viable cells of Lactobacillus. The formulations are useful for oral administration and treatment of infections of the gastrointestinal tract caused by pathogens.

WO2007/073709 discloses novel strains of Lactobacillus and their use against infections by H. pylori. In contrast to known Lactobacillus strains, the novel strains are capable of aggregating H. pylori under physiological conditions. The aggregates seem to be formed, because the Lactobacillus cells bind to the H. pylori and induce the formation of mixed aggregates. The relatively large aggregates are not capable of entering or penetrating the mucus any more. Consequently, the aggregated cells are not capable any more of contacting the epithelial cells of the stomach and infecting them. The aggregates are not attached to the inner walls of the gastrointestinal tract. They accumulate in the lumen, pass the gastrointestinal tract and are secreted naturally. The overall level of H. pylori is reduced and inflammatory reactions of the immune system are healed or prevented. Specifically, infections caused by H. pylori, such as gastritis and ulcers, are treated or prevented.

These novel strains described by WO2007/073709 have a different mode of action and also a different efficiency compared to previously known Lactobacillus strains useful against H. pylori. Strains known in the art fail to form aggregates with H. pylori. Generally, known strains exert a beneficial effect against H. pylori by competing with H. pylori cells in the stomach and thereby slowly replacing the cells. The rapid formation and secretion of aggregates thus provides a novel approach in the treatment of H. pylori infections.

The examples of WO2007/073709 provide evidence that co-aggregation occurs in an assay with buffered bacterial solutions and artificial stomach juice. However, it would be desirable to provide the Lactobacillus in a form, which is more acceptable to the consumer. Co-aggregation of two different bacterial strains is a complex process and it cannot be assumed that the reaction would also be efficient in other environments.

There is a basic need to provide Lactobacillus compositions, which are more acceptable to the consumer, but nonetheless do not interfere with the desired co- aggregation and are thus efficient against H. pylori. Since H. pylori infections are widespread, it is also desirable to provide efficient means for prevention and treatment, which are available easily and at low costs also for large numbers of individuals.

### Problem underlying the invention

The problem underlying the invention is to provide novel compositions, methods and uses which overcome the above mentioned problems and which are highly efficient in the treatment or prevention of diseases associated with H. pylori.

It is a specific problem underlying the invention to provide food compositions, which are highly effective against H. pylori. The level of H. pylori cells in the gastrointestinal tract of an animal, especially a human, shall be reduced rapidly and extensively. Further, the number of H. pylori cells in the stomach and intestine shall be maintained at a low level. The composition shall not have disadvantageous physiological side effects and shall be pharmacologically acceptable. Further, the taste of the composition shall be agreeable to the consumer, such that regular consumption is not constrained. The composition shall be manufactured and stored conveniently and at relatively low costs. Thus, a simple, cheap and efficient treatment of large numbers of individuals shall be possible.

### Disclosure of the invention

Surprisingly, the problem underlying the invention is solved by the use of skim milk as a carrier for Lactobacillus or a Lactobacillus preparation, which comprises *Lactobacillus* cell walls, cell wall fragments and/or cell wall constituents for the aggregation of *Helicobacter pylori,* wherein the *Lactobacillus* or a *Lactobacillus* preparation is capable of aggregating *Helicobacter pylori* under physiological conditions. The Lactobacillus may be dead or viable. In a specific embodiment of the invention, the Lactobacillus or a major portion thereof is dead. It is assumed that the ability of the Lactobacillus to aggregate H. pylori is mediated by the cell walls of the Lactobacillus, and that cell walls or dead cells effectively aggregate H. pylori. Thus, in a specific embodiment, the Lactobacillus is a Lactobacillus preparation, which comprises or consists of Lactobacillus cell walls, cell wall fragments and/or cell wall constituents. Preferably, the Lactobacillus preparation comprises membranes and/or membrane fragments. For example, the Lactobacillus preparation may be a spray-dried Lactobacillus preparation. Such a spray-dried preparation comprises dead cells and a carrier, such as carbohydrates, such as dextrin. Accordingly, it was found that spray- dried Lactobacillus is highly efficient in the inventive food composition, and that the carrier does not inhibit the co-aggregation reaction. Spray-dried cells are advantageous, because they can be handled conveniently by food industry and consumers when preparing the food composition. Spray dried Lactobacillus is commercially available under the trademark Pylopass from Lonza AG, CH. Alternatively, cell walls can be isolated by routine methods known in the art. For example, cell walls can be obtained by cell disruption and/or lysis and subsequent centrifugation. In other embodiments, at least 10 percent of the total Lactobacillus cells, preferably more than 50 percent or more than 90 percent are living cells. In another embodiment, essentially all the cells are viable, i.e. more than 95 percent or more than 99 percent of the total Lactobacillus cells. The food composition is thus a probiotic. In a preferred embodiment, the food composition is an end product, which is ready for consumption by a consumer. It thus can be bought, or obtained otherwise, by the consumer. However, the food composition may also be a basic component for the production of other foods. In a preferred embodiment of the invention, the Lactobacillus is selected from Lactobacillus reuteri, Lactobacillus fermentum, Lactobacillus brevis and Lactobacillus pentosus.

The Lactobacillus is capable of aggregating H. pylori under physiological conditions. Preferably, the Lactobacillus capable of aggregating H. pylori is a strain disclosed by WO 2007/073709, which is hereby expressively. As already outlined above, this document discloses Lactobacillus strains which are capable of binding and aggregating Helicobacter pylori cells in the gastrointestinal tract under physiological conditions. The aggregates are not capable of entering or passing the mucus anymore and are secreted, thereby preventing an inflammatory reaction of the immune system. The Lactobacillus strains are thus highly efficient against diseases associated with H. pylori, such as gastritis or ulcers.

The strains are usable for preventing and/or treating an infection with H. pylori. Even when an infection has already occurred, the Lactobacillus prevents further infection with H. pylori bacterial cells and the existing infection can be treated more easily by elimination of the H. pylori, which have already passed the mucus. The elimination of cells, which have already caused the infection, is supported or mediated by the natural immune response of the individual. In addition, it is assumed that the Lactobacillus strains disclosed by WO 2007/073709 are also effective in inhibiting urease activity of H. pylori. Thereby, H. pylori cells within the formed aggregates lose their protection against gastric acid, which renders their elimination even more effective. The term "under physiological conditions" refers to the conditions, under which the H. pylori to be aggregated dwell (live). If H. pylori in the gastrointestinal tract are to be treated, then physiological conditions are those in the gastrointestinal tract, i.e. in the presence of gastric acid. Preferably, "physiological conditions" correspond to assay conditions with artificial gastric juice (AGJ; fresh solution of 5g/L NaCl solution, pH 4.0, plus 3 g/L pepsin). The pH of the artificial gastric juice can be adjusted with an appropriate acid, such as HCI.

The term "aggregate" refers to the formation of aggregates of bacteria, in which the bacterial cells bind or stick to each other. Such aggregates are visible under the microscope. Typically, the aggregates formed during aggregation have a diameter of approximately 1 to 10 pm, but they may be even larger in some instances. The aggregates comprise Lactobacillus and H. pylori cells and are thus co-aggregates. Auto-aggregation of Lactobacillus is not desired, because auto- aggregated Lactobacillus is not available any more for co-aggregation. A useful aggregation assay for testing the formation of aggregates is outlined in the working examples. Typically, culturing conditions are selected which mimic physiological conditions in the gastrointestinal tract of the respective animal, such as a human. A similar useful assay is disclosed in WO2007/073709 in examples 2 and 3 and on pages 9 to 12. Preferably, H. pylori is provided in artificial gastric juice. Lactobacillus is provided in carrier, such as milk. Then, the Lactobacillus in carrier is diluted with PBS buffer, preferably at a ratio of 1:10. The assay could also be carried out with a dilution of <1:10 or>1:10, but this is less preferred. Then, the H. pylori in artificial gastric juice is mixed at a ratio of 1:1 with the Lactobacillus in carrier/PBS. Other ratios of mixing>1:1 or <1:1 are also possible, but less preferred. Preferably, the amount of H. pylori which is co-aggregated is at least 10 percent, at least 50 percent or at least 100 percent higher with the inventive food composition, when compared to the amount of H. pylori which is auto-aggregated in the corresponding food composition without the Lactobacillus or Lactobacillus preparation capable of aggregating H. pylori.

The Lactobacillus is selected from Lactobacillus strains deposited as No. DSM 17648, DSM 17646, DSM 17647, DSM 17649, DSM 17650, DSM 17651, DSM 17652 or DSM 17653 at the DSMZ (Deutsche Sammlung von Mikroorganismen und Zellkulturen GmbH, Mascheroder Weg lb, 38124 Braunschweig, Germany). As outlined in WO 2007/073709, these strains are deposited for public use. The strains DSM 17646, 17649, 17652 and 17653 are Lactobacillus brevis. DSM 17650 is Lactobacillus pentosus. In a preferred embodiment of the invention, the Lactobacillus is DSM 17648. The strains DSM 17647, 17648 and 17651 are Lactobacillus fermentum. According to example 5 of WO 2007/073709, taxonomic determination of the strains was carried out by determining carbohydrate fermentation patterns according to the API50CH systems (BioMerieux, France). This test is based on the fermentation of 49 different carbohydrates and allows the identification of a strain as Lactobacillus fermentum with high certainty. In addition, the present inventors examined the strain DSM 17648 using a 16s DNA-based method. When using this approach, the strain was classified as Lactobacillus reuteri. More specifically, the strain was correlated with the L. reuteri type strain LMG9213T, but seems to be a different clone. Nowadays, the 16s DNA based method is considered more reliable than carbohydrate fermentation patterns. However, precise taxonomic determination of related strains of the same genus is generally difficult.

In a preferred embodiment of the disclosure, the amount of Lactobacillus in the composition is between 10⁴ and 10¹⁵, preferably between 10⁶ and 10¹³, more preferably between 10⁸ and 10¹² cells, most preferably between 10⁹ and 10¹¹ cells. Specifically, it was found that an appropriate amount of cells in a single daily dose is 2x10¹⁰. The total number refers to the total amount of living and dead cells. The number of cells can be determined by known methods, for instance with a cell counter. Reference is a single daily dose. However, reference can also be a packaging unit. If a Lactobacillus preparation is used, then the amount of cell walls, cell wall fragments and/or cell wall constituents used corresponds to the amounts of cell walls, cell wall fragments and/or cell wall constituents of whole cells. In other words, less cell walls or fragments or constituents thereof are required in the composition.

The term "carrier" suggests that the Lactobacillus is distributed throughout the carrier. Thus the carrier is the main component of the food composition. Preferably, the total amount of Lactobacillus in the carrier is below 5 percent (w/w), more preferably below 2 percent (w/w) or below 0.5 percent (w/w), based on the combined amount of carrier and Lactobacillus. In a specific embodiment, the food composition consists of the Lactobacillus and the carrier. However, the composition may comprise additives. Preferably, such additives are also distributed throughout the carrier.

According to the invention, the carrier is skim milk. The skim milk may be a liquid milk fraction, or may be obtained by dissolving skim milk powder in water or aqueous medium. Skim milk powder is generally obtained by withdrawing water from skim milk. Skim milk (skimmed milk is a fraction of whole milk, which is obtained when the fat (cream) is removed from whole milk. Milk fat is present in whole milk in the form of globules, which are composed mainly of triacylglycerols and are surrounded by a membrane consisting of complex lipids such as phospholipids, along with proteins. Milk fat lipids are 97-98 percent triacylglycerols, small amounts of di- and monoacylglycerols, free cholesterol and cholesterol esters, free fatty acids and phospholipids. Whole milk from cows comprises about 4 percent milk fat. Commercially available skim milk may still have a low milk fat content of up to 0.3 percent. According to the invention, the fat content of the skim milk and/or of the carrier is preferably below 0.5 percent (w/w), more preferably below 0.3 percent (w/w) or below 0.1 percent (w/w), most preferably about 0 percent (w/w). Throughout this application, all percent values relating to parts of compositions are in weight percent, unless specifically stated otherwise.

Surprisingly, it was found that co-aggregation of H. pylori with Lactobacillus is more efficient in skim milk carrier compared to a whole milk. Undesired auto- aggregation of Lactobacillus was found to be less pronounced in skim milk, and overall co-aggregation was better. Without being bound to theory, whole milk may comprise ingredients which interfere with the co-aggregation reaction, which are not present in skim milk.

In another embodiment of the disclosure, the carrier is semi skim milk. The semi skim milk and/or the carrier may then comprise between about 0.5 to about 2.5 percent (w/w) milk fat.

In another embodiment of the disclosure, the carrier is whole milk (full milk, milk). Whole milk is milk that contains all its constituents as received from the cow or other milk giving animal, i.e. it is milk, from which no constituent, such as fat, has been removed. Whole milk may be used without any thermal treatment. However, whole milk can also be used after pasteurization and/or homogenization. Alternatively, dry whole milk or whole milk powder can be used. Dry whole milk or whole milk powder is usually obtained by removing water from pasteurized, homogenized whole milk. Although whole milk is less efficient than skim milk, an inventive food composition with whole milk is capable of desired co-aggregation. Whole milk is thus a useful and applicable carrier in the inventive food composition. In this embodiment, the carrier may comprise between about 2.5 and about 6 percent (w/w), preferably between about 3 and 5 percent (w/w) milk fat. Semi skim milk and milk comprise skim milk as a fraction thereof. Overall, the carrier may comprise between 0 and 6 percent (w/w) milk fat. The milk may be UHT milk (ultra heat treated milk). The skim milk, semi skim milk or milk may be provided as a powder, which is re- dissolved in the composition. Preferably, the milk is cow milk. However, the milk may also be from other mammals, such as goats. The skim milk may be diluted, preferably with water or a buffer. In this embodiment, the carrier may comprise 10 to 95 percent (w/w), preferably 20 to 80 percent (w/w) skim milk.

The carrier is not derived from milk by a fermentation process. Preferably, the carrier was not fermented and/or does not comprise a fermented dairy (milk) product. Preferably, the composition does not comprise a fermented dairy product. In this respect, the Lactobacillus capable of aggregating H. pylori, which had been cultured, is not considered a dairy product. Specifically, the carrier does not comprise yogurt and/or whey, or fractions thereof. Some commercially available yogurts comprise skim milk or skim milk powder, which was fermented. Upon fermentation, especially by bacteria, the chemical composition and the structure change significantly. For example, lactose is converted into lactate. Preferably, the lactate content of the carrier was not reduced, when compared to the milk from which it was derived. Preferably, the inventive composition has a lactate content between 2 and 8 percent (w/w), more preferably between 3 and 7 percent (w/w) or between 4 and 6 percent (w/w). However, the Lactobacillus may be derived from a fermentation medium and the composition may thus comprise traces or small amounts of this fermentation medium. In a preferred embodiment of the invention, at least 50 percent, more preferably at least 90 percent or at least 98 percent of the H. pylori cells co-aggregate upon contact with the inventive composition, as determined in an aggregation assay carried out in vitro, preferably under the conditions outlined in the working examples. Preferably, less than 20 percent, less than 5 percent or less than 2 percent of the Lactobacillus auto-aggregates in a respective control assay without H. pylori.

The inventive use is preferably not administered on an empty stomach. The pH of gastric fluid in an empty stomach is typically in the range of 1 to 2. The pH of gastric fluid after a meal is typically between 2 to 6. According to the invention, it was found that the desired co-aggregation is inhibited at low pH, especially below pH 2.5. At a higher pH value, especially from pH 3.0 to 6.0, the co-aggregation is highly effective. Thus the use is especially for administration when the stomach pH is above 2.5, especially pH 3.0 or more, for example up to pH 6 or up to pH 5, for example from pH 2.5 to 7, or from pH 3 to pH 6. The disclosed composition is preferably administered during a meal and/or after a meal, preferably within 30 min, within 1 hour or within 2 hours after a meal.

In a preferred embodiment of the disclosure, the pH of the food composition is between 4 and 10, preferably between 5 and 8. The pH of fresh milk is about 6.5. In principle, it is advantageous that the pH of the composition is relatively high, such that consumption increases the pH of the gastric fluid. However, the pH may be chosen and adjusted such that the composition is stable upon storage. The inventive food composition may consist of the Lactobacillus, or Lactobacillus preparation, and the carrier. However, additives may be comprised, for example for providing a desired taste or functionality.

In a preferred embodiment of the disclosure, the composition comprises at least one additional sugar. As used herein, the term "sugar" refers to carbohydrates which function as sweeteners. The sugar is "additional", because it is not introduced into the composition as a component of the skim milk. According to the invention, it was found that the inventive food composition is capable of aggregating H. pylori in the presence of sugar, even if large amounts of sugars of about 16 percent (w/w) are included. This was an important finding, because sweetened food compositions based on dairy products are often more acceptable to the consumer. It was also unexpected, because a sugar content of about 16 percent (w/w) significantly changes the environment of the H. pylori and Lactobacillus.

The additional sugar may be a low molecular weight compound, such as glucose, lactose, fructose, galactose or sucrose. The additional sugar may also be, or comprise, a polymeric compound, such as dextrins, preferably nutriose. The additional sugar could also be added as a mixture of sugars, such as maltodextrins, preferably Maltodextrin DE19, or glucose syrup. The additional sugar may be added as part of an additive having a sugar content, preferably a high sugar content, for example an additive comprising more than 50 percent (w/w), more than 80 percent (w/w) or more than 90 percent (w/w) sugar. For example, honey, or a syrup, such as corn syrup, maple syrup, sugar beet syrup, glucose syrup or fructose syrup, or a fruit juice concentrate may be added.

In highly preferred embodiments of the disclosure, the additional sugar is selected from glucose, lactose, fructose, dextrin and maltodextrin, or is provided in the form of honey. It was found that these sugars or honey do not significantly inhibit aggregation of H. pylori when present in the inventive food composition in combination with a skim milk carrier. In preferred embodiments, the additional sugar is selected from galactose, sucrose, nutriose and glucose syrup. It was found that these sugars inhibit aggregation of H. pylori only moderately when present in combination with a skim milk carrier. The additional sugar is added in an amount to provide a desired degree of sweetness. For example, up to 25 percent (w/w) or up to 20 percent (w/w) sugar may be added. The amount of additional sugar may be from 0.1 to 25 percent (w/w) or from 0.5 to 20 percent (w/w) of the food composition. In order to achieve sweetness comparable to commercial dairy products, between 10 to 20 percent (w/w), preferably about 16 percent (w/w) additional sugar may be added. In another embodiment, the composition comprises a sugar alcohol, such as sorbitol, isomalt, maltitol, mannitol, lactitol and xylitol. However, it was found that the aggregation of H. pylori, although not entirely inhibited, is reduced in the presence of sorbitol. In another embodiment, the composition comprises a sweetener, which is not a saccharide (sugar). Common non-sugar sweeteners are stevia, aspartame, sucralose, neotame, acesulfame potassium and saccharin.

In another embodiment, other microorganisms or compounds with activity against Helicobacter pylori are added. Alternatively or in addition, other microorganisms or compounds, which are beneficial to the individual for other reasons, may be included, such as vitamins. The food composition may comprise other typical ingredients for improving taste, flavour, stability and the like. Such ingredients may be fruits or fruit preparations, flavour additives, preservatives, and the like. In specific embodiments, the food composition may comprise up to 25 percent (w/w), up to 10 percent (w/w) or up to 5 percent (w/w) additives. Preferably, the additives are distributed throughout the carrier.

When adding such additives, it should be checked in an aggregation assay that the efficiency of the Lactobacillus is not decreased in such a mixture. Thus, additional ingredients should be selected which do not to interfere with the Lactobacillus or inhibit the Lactobacillus. Specifically, the additives should not increase auto-aggregation of Lactobacillus or decrease co-aggregation with H. pylori.

The carrier is preferably a liquid. Thus, the composition is preferably a drink. In a preferred embodiment of the disclosure, the food composition comprises or consists of
(A) 60 to 96 percent milk, preferably skim milk,
(B) 0.01 to 0.5 percent Lactobacillus or Lactobacillus preparation,
(C) 0 to 25 percent, preferably 10 to 20 percent, additional sugar
(D) 0 to 20 percent additives, wherein the total of components (A) to (D) is 100 percent (all percentages in weight percent), wherein the milk, preferably skim milk, may be obtained by dissolving milk or skim milk powder in water or aqueous medium.

In a preferred embodiment of the disclosure, the carrier comprises or consists of
(a) 2 to 8 percent proteins
(b) 0 to 6 percent, preferably 0 to 0.5 percent milk fat
(c) 2 to 8 percent lactose
(d) 0 to 25 percent carbohydrates different from lactose
(e) 0.1 to 2 percent minerals
(f) 0 to 4 percent other dry matter and
(g) 60 to 96 percent water, wherein the total of components (a) to (g) is 100 percent (all percentages in weight percent).

Subject of the invention is the Use of skim milk as a carrier for Lactobacillus or a Lactobacillus preparation, which comprises *Lactobacillus* cell walls, cell wall fragments and/or cell wall constituents for the aggregation of *Helicobacter pylori,* wherein the *Lactobacillus* or a *Lactobacillus* preparation is capable of aggregating *Helicobacter pylori* under physiological conditions.

The inventive use may be administeration to animals suffering from H. pylori infections, or suspected of being suffering from H. pylori infections, or for preventing such infections. Preferably, the animal is a mammal, more preferably human. The animals may also be non-human, for example a pet, such as a cat or dog, or a companion animal, such as cattle, horses, pigs or sheep.

Another subject of the disclosure is a method for producing a food composition capable of aggregating Helicobacter pylori, comprising the steps: a) providing Lactobacillus, or a Lactobacillus preparation which comprises Lactobacillus cell walls, cell wall fragments and/or cell wall constituents, which is capable of aggregating Helicobacter pylori under physiological conditions, b) providing a carrier, wherein the carrier comprises skim milk, and c) mixing the Lactobacillus or Lactobacillus preparation and the carrier to obtain the food composition.

In this production method, before, during or after step c), further ingredients may be added. Preferably, Lactobacillus is initially cultivated in an appropriate fermentation medium. Conditions for growing Lactobacillus are known in the art and for example disclosed in WO 2007/073709. Preferably, the cells are separated from the cultivation medium by centrifugation, optionally washed and resuspended in a desired solution, for example water or buffer. Washing and centrifugation steps may be repeated in order to avoid transfer of fermentation medium into the food composition. The capability of the cells and compositions for aggregating H. pylori may be routinely checked in aggregation assays during the production process. The cells may be added directly to the food composition or preferably dried in an appropriate matrix before being added to the food composition. This way the activity of the Lactobacillus preparation can be standardized.

Also disclosed is a method for reducing the level of Helicobacter pylori in the gastrointestinal tract of an animal, comprising administering to the animal an inventive food composition. In other words, the method is for aggregating H. pylori in the gastrointestinal tract of an animal and/or secreting H. pylori from the gastrointestinal tract of an animal.

Also disclosed is a medicament for reducing the level of Helicobacter pylori in the gastrointestinal tract of an animal. The medicament is any composition as described above. Preferably, the medicament is a food composition. The composition of the medicament corresponds to the food composition described above. Therefore, the medicament is a composition comprising Lactobacillus, or a Lactobacillus preparation which comprises Lactobacillus cell walls, cell wall fragments and/or cell wall constituents, which is capable of aggregating H. pylori under physiological conditions, and a carrier, wherein the carrier comprises skim milk. Preferably, the carrier is skim milk. The medicament is for treating and/or preventing infections by H. pylori, especially infections of the gastrointestinal tract. Preferably, the medicament is used for treating gastritis, gastric ulcers or stomach cancer. Specific embodiments of the medicament are those described in the claims and specific embodiments above for the food composition. The medicament may comprise further ingredients typical for such medicaments, such as other active agents.

The food composition solves the above-mentioned problems. Surprisingly, it was found that skim milk or a dairy product comprising skim milk is applicable as a carrier for Lactobacillus. It could not be expected that a complex co-aggregation of two bacterial strains would be efficient in such a complex carrier. In line with this, and as shown in the examples, most dairy products are not suitable carriers for food compositions comprising Lactobacillus for aggregating H. pylori. In carriers such as yogurt or whey, either Lactobacillus auto-aggregates or there is no co-aggregation. The invention provides a simple and efficient composition and method for preventing and treating infections associated with or caused by H. pylori colonization of the gastrointestinal tract. The inventive carrier is available in large amounts, can be produced and stored at low costs, and is highly acceptable to consumers. Therefore, the composition can also be provided to large numbers of individuals, who do not have access, or only limited access, to medical care.

### Figures:

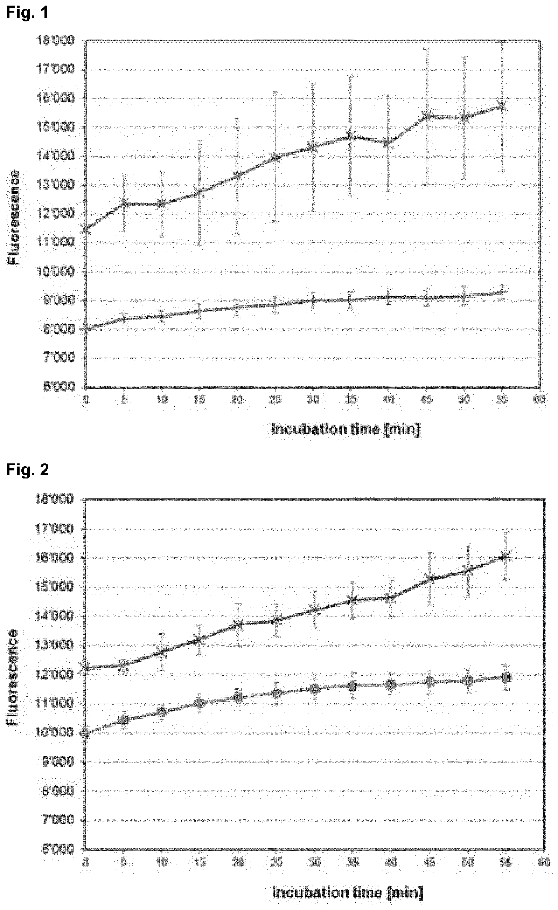

Figure 1 shows the results of the dynamic assay according to example 35 in graphic form. Overall fluorescence (485/535 nm) determined during the aggregation reaction with skim milk carrier is shown dependent from incubation time (minutes). The upper graph (x) corresponds to the co-aggregation probe with H. pylori and L. reuteri. The lower graph (I) corresponds to auto-aggregation of the blind control with H. pylori in the absence of L. reuteri.
Figure 2 shows the results of the dynamic assay according to example 36 in graphic form. Overall fluorescence (485/535 nm) determined in the aggregation reaction with full milk carrier is shown dependent from incubation time (minutes). The upper graph (x) corresponds to the co-aggregation probe with H. pylori and L. reuteri. The lower graph (·) corresponds to auto-aggregation of the blind control with H. pylori in the absence of L. reuteri.

### Examples:

All percent values relating to parts of compositions are provided in weight percent, unless specifically stated otherwise.

### Strains and cell cultures

A working cell culture (WCC) of H. pylori DSM 21031 was prepared by growing the strain on Brucella medium supplemented with 5 percent defibrinated horse blood (Oxoid) at 37 degrees centigrade under microaerophilic conditions. The horse blood was pre-treated by freezing at -20 degrees centigrade and then thawing to ensure the lysis of the blood cells, which release their growth factors into the medium. At the end of the growth phase the culture was mixed with sterile glycerol to achieve an end concentration of 50 percent. This working cell culture was left for 2 - 3 hours at room temperature before storing at -80 degrees centigrade This WCC (500 µl) was used to inoculate Brucella medium supplemented with 10 percentfoetal calf serum which was incubated at 37 degrees centigrade for 72 h. The active test culture was characterized by motile, curved to spiral rods. If the culture was characterized by the coccoid, non-motile form then this culture was discarded as it is not suitable for the aggregation test. After growth a solution of H. pylori with an optical density of 2.0 (A = 600 nm) was prepared by centrifuging the culture at 4500 g for 5 min. The supernatant was discarded and the cells washed in phosphate buffered saline, pH 7.0 (PBS). The pellet was carefully re- suspended in this buffer and then centrifuged again at 4500 g for 5 min. This supernatant was discarded and the pellet was carefully re-suspended in artificial gastric juice (AGJ; fresh solution of 5g/L NaCl solution, pH 4.0 plus 3 g/L pepsin). It is important that solution of artificial gastric juice and H. pylori is prepared just prior to the aggregation test.

A fresh culture of L. reuteri DSM17648 (classified as L. fermentum in WO2007/073709 by carbohydrate patterns), which is capable of aggregating with H. pylori (positive control) was prepared. A culture was grown on MRS medium in closed tubes overnight at 37 degrees centigrade without shaking. Analogue to the harvesting of H. pylori (described above) the cells were centrifuged, the pellet washed in PBS, re-centrifuged and then the pellet was re-suspended in PBS instead of artificial gastric juice to achieve solutions with an optical density of 4.0 (A = 600 nm).

### Aggregation assays

An aggregation test in artificial stomach juice was used according to WO2007/073709, which is based on mixing L. reuteri in phosphate buffered saline (PBS; pH 7.5) with Helicobacter pylori in artificial stomach juice (pH 4.0). In some examples, the test was modified by altering the pH of the artificial gastric juice to determine the effect of pH of the L. reuteri solution on aggregation. In the test, undesired auto-aggregation and desired co-aggregation were examined, respectively. The samples were mixed carefully and aggregation was monitored after about 10 min at room temperature. The co-aggregation is judged both macroscopically and microscopically (400 times magnification). The aggregation results were classified as: ++++ = very strong; +++ = strong; ++ = some; + = little; - = none nd = not determined.

### Comparative examples 1 to 7: Effect of pH on aggregation without carrier

In the following examples, aggregation test according to WO2007/073709 were carried out as outlined above with strains as prepared above. In example 7, the test protocol of WO2007/073709 was applied. The resulting mixture had a pH of about 5.5 and good co-aggregation was observed. In examples 1 to 6, the test was modified to determine the effect of pH of the L. reuteri solution on aggregation. Experiments covered a pH range from 1.5 to 6.0 of the artificial stomach juice, reflecting the differences in pH between a "starved" stomach at low pH and a stomach after consuming food having a high pH. The negative controls were also carried out. Results are not reported herein as in all cases they were negative for co-aggregation as expected. The buffer was used to prepare the L. reuteri solution for the test, which was combined with the artificial stomach juice (pH 4) comprising H. pylori. The materials and results are summarized below in table 1 (upper panel). The results show that the co-aggregation is not observed at pH of 2.5 or lower, whereas good co-aggregation is observed between pH 3 to 6.

### Comparative examples 8 to 14: Whey carrier

The effect of whey as a carrier on the co-aggregation test was tested at pH 3 to 6. Experiments 8 to 14 were carried out together with experiments 1 to 7, which are thus valid controls. The whey was a commercially available dairy product (trademark Yuma, produced by Mercola, CH) and had the following contents per 100 g powder: 12 g protein, 74 g carbohydrate, 1.2 g fat, 890 mg calcium, 180 mg magnesium, 570 mg phosphorous, 2.4 g potassium, vitamins. The whey was dissolved at 5 percent (w/w) in the appropriate buffer and this carrier was used to prepare the L. reuteri solution for the test. The effect of the buffer with whey was tested on the co-aggregation ability of the L. reuteri strain with H. pylori. The negative control results are not reported here as in all cases they were negative for co-aggregation as expected. The materials and results are summarized in table 1. The results show that co-aggregation does not occur at any pH. This shows that whey, even in relatively small amounts, inhibits co-aggregation and is not an appropriate carrier.

**Table 1: Comparative examples 1 to 7 (upper panel): pH dependent auto- aggregation and co-aggregation without carrier; comparative examples 8 to 14 (lower panel): pH dependent co-aggregation and auto-aggregation with whey carrier.**

| **Example** | **1** | **2** | **3** | **4** | **5** | **6** | **7** |
|---|---|---|---|---|---|---|---|
| buffer | citrate pH 1.5 | citrate pH 2.5 | citrate pH 3.0 | citrate pH 4.0 | citrate pH 5.0 | succinate pH 6.0 | PBS pH 7.5 |
| carrier | - | - | - | - | - | - | - |
| co-aggregation | - | - | ++ | +++/+++ | +++ | ++ | + |
| auto-aggregation | - | - | - | ++/- | - | - | - |
| pH of test sample | 2.06 | 2.57 | 3.0 | 4.5 (4.2) | nd | nd | 5.5 |
| | | | | | | | |

| **Example** | **8** | **9** | **10** | **11** | **12** | **13** | **14** |
|---|---|---|---|---|---|---|---|
| buffer | citrate pH 1.5 | citrate pH 2.5 | citrate pH 3.0 | citrate pH 4.0 | citrate pH 5.0 | succinate pH 6.0 | PBS. pH 7.5 |
| carrier | whey | whey | whey | whey | whey | whey | - |
| co-aggregation | nd | nd | - | - | - | - | + |
| auto-aggregation | nd | nd | - | - | - | - | - |
| pH in test | nd | nd | 4.0 | 5.0 | nd | nd | 5.5 |

### Comparative Examples 15 to 27: Yogurt and yogurt serum carriers

Aggregation assays as outlined above were carried out with different commercially available yogurts as carriers and L reuteri suspended in PBS buffer. Auto- aggregation of L. reuteri was observed in the control experiments without H. pylori (data not shown).

Thus it was examined whether yogurt serum alone would be an appropriate carrier with artificial stomach juice (ASJ) at a pH ranging from 1.5 to 4.0. Yogurt serum was prepared from commercially available natural yogurt (trademark "Natural yogurt classic", Migros, CH; manufactured from skim milk and cream; comprising 3.6 g fat/100ml, 3.5 g protein/100ml, 5 g carbohydrate/100ml, minerals and vitamins). Yoghurt serum (pH 4.08) was prepared by centrifuging the natural yoghurt and using the clear serum in the test. Aggregation experiments in yogurt serum/PBS and controls in PBS buffer comprising L. reuteri were carried out. The results are summarized in table 2 below. The results show that at the relevant pH between 1.5 and 4, there is a tendency towards auto-aggregation in the yogurt serum carrier, which is not observed to the same extent in PBS buffer. Therefore, in a yogurt serum carrier and at the relevant pH between 1.5 and 4, L. reuteri seems to be capable of co-aggregation, but the positive effect is diminished at least partially by auto-aggregation.

**Table 2: Comparative examples 21 to 26: co-aggregation and auto-aggregation with yogurt carrier with control experiments 15 to 20 without yogurt serum.**

| **Example** | **15** | **16** | **17** | **18** | **19** | **20** |
|---|---|---|---|---|---|---|
| carrier | - | - | - | - | - | - |
| pH of artificial stomach juice | 1.5 | 2.0 | 2.5 | 3.0 | 3.5 | 4.0 |
| test pH | nd | nd | nd | nd | nd | nd |
| co-aggregation | - | (+) | +++ - ++++ | +++ | +++ | +++ |
| auto-aggregation | - | - | (+) | - | - | - |
| | | | | | | |

| **Example** | **21** | **22** | **23** | **24** | **25** | **26** |
|---|---|---|---|---|---|---|
| carrier | yogurt serum | yogurt serum | yogurt serum | yogurt serum | yogurt serum | yogurt serum |
| pH of artificial stomach juice | 1.5 | 2.0 | 2.5 | 3.0 | 3.5 | 4.0 |
| test pH | 3.73/3.85 | 3.95 | 4.05/4.02 | 4.06 | 4.09 | 4.08/4.06 |
| co-aggregation | +++/+++ | +++ | +++/+++ | ++ | ++ | ++/+++ |
| auto-aggregation | +/++ | ++ | +/++ | ++ | ++ | +/++ |

An additional example 27 was carried out with fruit yogurt serum. Fruit yoghurt serum (pH 4.07) was prepared as described above from strawberry yoghurt (trademark Migros Bio). This yoghurt was produced from full milk, skim milk powder, strawberries (8.8 percent) and 10.7 percent sugar (yogurt comprising 3.4 g fat/100 ml, 3.5 g protein/100 ml, 16 g carbohydrate/100 ml). The fruit yogurt serum was tested in an assay according to example 26. No co-aggregation and no auto-aggregation were observed under the test conditions. The fruit yoghurt serum showed a different effect compared to natural yoghurt serum, inhibiting completely the co- aggregation with H. pylori.

### Examples 28 to 34: Skim milk carrier and sugar addition

The effects of skim milk (10 percent w/w solution of skim milk powder in PBS, or 100 percent skim milk, pH 6.5), and four different sugars (16 percent solution of glucose, galactose, fructose or lactose in PBS), alone and in combination, were examined. Comparative example 29 is another control with natural yogurt serum at pH 4.2. The conditions and results are summarized in table 3 below. Surprisingly, skim milk neither induces significant auto-aggregation nor inhibits co-aggregation. The results show also that the sugars do not affect co-aggregation and do not induce auto-aggregation. Control experiments were carried out, in which natural yogurt serum was added to each sample. The results of the co-aggregation tests with skim milk and different sugars show the same degree of aggregation as with the control, which indicates that skim milk, as well as the added sugars, have no negative effect on the aggregation at the concentrations tested. Again, co- aggregation is also observed in natural yogurt serum, but auto-aggregation is relatively strong.

**Table 3: Comparative examples 28 to 32: co-aggregation and auto-aggregation with various sugars and natural yogurt serum; inventive examples 33 and 34: with skim milk carrier.**

| **Example** | **28** | **29** | **30** | **31** | **32** | **33** | **34** |
|---|---|---|---|---|---|---|---|
| carrier | PBS pH 7.5 | yogurt serum pH 4.2 | PBS pH 7.5 | PBS pH 7.5 | PBS pH 7.5 | PBS skim milk powder (10%) | skim milk pH 6.5 |
| sugar additive | - | gluc | gal | lac | fruct | - | - |
| co-aggregation | +++ | +++ | +++ | +++ | ++ | +++ | +++ |
| auto-aggregation | - | ++ | - | - | - | - | + |

Note: control experiment 26: L. reuteri + PBS, pH 7.5 mixed with H. pylori + artificial stomach juice (AJS, pH 4.0); glue = glucose, gal = galactose; lac = lactose; fruct = fructose. In an additional series of experiments, it was checked whether the aggregation is also efficient in the presence of skim milk in combination with an additional sugar, sugar alcohol or a natural product comprising sugar. Aggregation assays were carried out as described above in the presence of 10 percent skim milk powder, and additional 16 wt percent of sugar, sugar alcohol or sugar-containing additive (honey or orange juice), respectively. It was found that aggregation of H. pylori glucose in the presence of a skim milk carrier is at the same level in the presence of additional lactose, fructose, maltodextrin or honey, when compared to the corresponding composition without additional sugar. Thus, these additives do not affect aggregation at all. Galactose, sucrose, nutriose and glucose syrup inhibit aggregation of H. pylori moderately in the presence of a skim milk carrier, but overall aggregation is still efficient. In the presence of sorbitol and skim milk carrier, aggregation of H. pylori is reduced, although not entirely inhibited. In the presence of orange juice, the level of aggregation was similar to the level of auto- aggregation.

### Examples 35 and 36: Comparison skim milk and whole milk

The following examples were carried out in order to determine if the advantageous effects as a carrier are specific for skim milk or found also for whole milk. For a quantitative comparison of skim milk and whole milk carrier, a fluorescence assay was used.

### Strains and cell cultures

Strains and cultures were prepared according to the general procedure described above for examples 1 to 34. In addition, the H. pylori was stained with a fluorescent marker as described in the following. Prior to the re-suspension of H. pylori in artificial gastric juice, the 10 ml stock solution of H. pylori cells was stained with 50 µl of a fresh prepared 1 mM 5(6)-CFDA-SE (5-(and-6)-carboxyfluorescein succinimidyl ester; Invitrogen, #V12883) fluorescence working solution based on phosphate buffered saline (PBS; pH 7.5). After incubating this blend during 30 min at 37 degrees centigrade in the darkness, the stained suspension was centrifuged at 4500 g for 5 min. The supernatant was discarded and the cells were washed in phosphate buffered saline, pH 7.5 (PBS). Just prior to the aggregation test, the pellet was carefully re-suspended in 10 ml artificial gastric juice as described above for examples 1 to 34.

The L. reuteri DSM 17648 suspension was prepared from spray-dried cells (Pylopass™, Lonza AG, comprising dextrin as spray-drying excipient) by the following procedure. 2 g cells were resuspended in the matrix (PBS, pH 7.5, milk or skim milk). This resuspended suspension contained 12 g dextrin per 100 ml suspension due to the composition of the spray dried L. reuteri cells. This suspension was prepared 30 min before use in the co-aggregation test (Swelling time). For the co-aggregation test, a dilution of 1:10 in PBS, pH 7, was prepared in order to minimize the matrix effect in the assay and to simulate a typical dilution comparable to the concentrations in the stomach. Thereby, the same concentration of cells (optical density of 4, at 600 nm, 1 cm) was adjusted as described above for examples 1 to 34, where PBS has been used instead of milk matrix.

### Co-aggregation assays

Aggregation and co-aggregation assays were carried out as described above for examples 1 to 34. In addition, co-aggregation was determined in fluorescence assays. The difference of fluorescence values of samples comprising H. pylori and L. reuteri compared to samples with H. pylori only gives an indication which amount of H. pylori has been co-aggregated. Co-aggregation was determined in a static assay and a dynamic assay. The respective samples are examined in 96- well plates by fluorescence spectroscopy with the following parameters:

| | |
|---|---|
| Mode: | Fluorescence Top Reading in 96-well plates with black bottom |
| Excitation wavelength: | 485 nm |
| Emission wavelength: | 535 nm |
| Excitation bandwidth: | 9 nm |
| Emission bandwidth: | 20 nm |

In the static assay, co-aggregation was determined by comparing fluorescence in the supernatants of the samples. Fluorescence values are lower in case of co- aggregation of H. pylori with L. reuteri compared to controls without L. reuteri, because less H. pylori is in the supernatant The evaluation of the results was carried out on a semi-quantitative base by calculating the relative differences of the fluorescence values and classifying them with respect to the known co- aggregation strengths: (+++) = very strong, (++) = strong, (+) = some; (+/-) = little and (-) = none, (nd) = not determined.

In the dynamic assay, co-aggregation was determined directly in a 96-well plate by measuring overall fluorescence of the samples. If co-aggregation occurs, fluorescence is higher compared to blind probes with H. pylori only without co- aggregation. The effect is observed because sedimentation of the co-aggregates results in higher fluorescence due to less turbidity of the sample.

### Results examples 35 and 36

A static assay as described was carried out for determining co-aggregation of H. pylori with L. reuteri in skim milk and ultra heat treated (UHT) full milk as carriers. As a control, auto-aggregation of L. reuteri was determined in the absence of H. pylori. The results are summarized in table 1 below. Strong co- aggregation was observed with both carriers. Sedimentation of the co-aggregates was macroscopically visible. Skim milk had no significant effect on auto- aggregation of L reuteri, but use of full milk coincided with strong auto- aggregation of L. reuteri.

**Table 1: Comparative examples 35 and 36: Co-aggregation and auto-aggregation with skim milk compared to full milk (UHT)**

| **Example** | **35** | **36** |
|---|---|---|
| Carrier | Skim milk, 1:10 in PBS pH 7.5 | Full milk 1:10 in PBS pH 7.5 |
| Additive | Dextrin | Dextrin |
| Co-aggregation | +++ | +++ |
| Auto-aggregation | + | ++ |

Dynamic assays were carried out as described above with skim milk and whole milk carriers. The results are shown in graphic form in figures 1 and 2. The overall fluorescence (485/535 nm) determined in the aggregation reaction with skim milk carrier is shown dependentfrom the incubation time in minutes. Figure 1 shows the results of example 35. The upper graph (x) corresponds to co-aggregation probe with H. pylori and L. reuteri cells. The lower graph (I) is a control, in which auto-aggregation of H. pylori is observed in the absence of L. reuteri. Figure 2 shows the results of example 36. The upper graph (x) shows results for the co- aggregation probe with H. pylori and L. reuteri cells. The lower graph (·) corresponds to auto-aggregation in the blind control with H. pylori in the absence of L. reuteri. Co-aggregation was slightly more pronounced with skim milk carrier than with whole milk carrier. Auto-aggregation of the control without L. reuteri was stronger with full milk compared to skim milk. However, in both cases sedimentation of H. pylori was increased by formation of co-aggregates in the presence of L. reuteri. Overall, the dynamic assay confirmed the results of the static assay.

In further experiments, the effect of UHT full milk carrier was compared to PBS, pH 7.5, carrier in co-aggregation assays. Less sedimentation of H. pylori was observed in full milk carrier when compared to PBS carrier (data not shown). It is assumed that the difference of the fluorescence values was triggered by the sedimentation of H. pylori, which results in lower fluorescence in the supernatant

Additional control experiments were carried out to ensure that the additive dextrin, which is present in spray dried L. reuteri probes, does not inhibit the co- aggregation reaction. In direct comparative experiments between fresh cells of L. reuteri and spray dried cells (trademark Pylopass), comparable co-aggregation was observed (data not shown). Therefore, it can be excluded that dextrin affects the co-aggregation assays.

In summary, examples 35 and 36 provide evidence that the skim milk carrier has a better overall performance than the full milk carrier with respect to specific co- aggregation of H. pylori with L. reuteri in artificial stomach juice. Skim milk is therefore the preferred carrier for dairy applications. The experiments also provide evidence that whole milk and skim milk slightly inhibit co-aggregation of L reuteri and H. pylori in artificial stomach juice. The effect is assumed to be the result of slightly increased auto-aggregation of L. reuteri with components in milk and skim milk.

## Claims

1. Use of skim milk as a carrier for Lactobacillus or a Lactobacillus preparation, which comprises *Lactobacillus* cell walls, cell wall fragments and/or cell wall constituents for the aggregation of *Helicobacter pylori,* wherein the *Lactobacillus* or a *Lactobacillus* preparation is capable of aggregating *Helicobacter pylori* under physiological conditions.

## Patentansprüche

1. Verwendung von Magermilch als Träger für Lactobacillus oder eine Lactobacillus-Zubereitung, die Lactobacillus-Zellwände, Zellwandfragmente und/oder Zellwandbestandteile zur Aggregation von *Helicobacter Pylori* enthält, wobei der *Lactobacillus oder eine Lactobacillus-Zubereitung* fähig ist *Helicobacter Pylori* unter physiologischen Bedingungen zu aggregieren.

## Revendications

1. Utilisation du lait écrémé comme support pour des Lactobacilles ou une préparation de Lactobacilles, qui comprend des parois cellulaires des *Lactobacilles,* des fragments de parois cellulaires et/ou des constituants de parois cellulaires pour l'agrégation d'*Helicobacter pylori,* dans laquelle les *Lactobacilles* ou une préparation de *Lactobacilles* est capable d'agréger des *Helicobacter pylori* dans des conditions physiologiques.
